(19) Europäisches Patentamt
European Patent Office
Office européen des brevets

(11) Publication number: 0 089 120
B1

(12) EUROPEAN PATENT SPECIFICATION

(45) Date of publication of patent specification: 22.07.87

(51) Int. Cl.⁴: A 61 K 7/00, A 61 K 7/32, A 61 K 7/48

(21) Application number: 83300808.9

(22) Date of filing: 17.02.83

# (54) Cosmetic composition.

(30) Priority: 18.02.82 IT 6718082

(43) Date of publication of application:
21.09.83 Bulletin 83/38

(45) Publication of the grant of the patent:
22.07.87 Bulletin 87/30

(84) Designated Contracting States:
AT BE CH DE FR IT LI NL SE

(56) References cited:
EP-A-0 013 390
EP-A-0 024 365

SEIFEN- ÖLE - FETTE - WACHSE, vol. 96, no. 14, 8th July 1970, page 493-495, Augsburg, DE K. BERGWEIN: "Kosmetik-Sticks"

CHEMICAL ABSTRACTS, vol. 76, no. 16, 17th April 1972, page 296, no. 89928y, Columbus, Ohio, US N. PILPEL: "Metal stearates in pharmaceuticals and cosmetics"

(73) Proprietor: UNILEVER NV
Burgemeester s'Jacobplein 1 P.O. Box 760
NL-3000 DK Rotterdam (NL)

(72) Inventor: Caserio, Domenico
Via Astico 13
I-20131 Milano (IT)
Inventor: Mignini, Elio
Via Gabbro 19
I-20161 Milano (IT)

(74) Representative: Tonge, Robert James et al
UNILEVER PLC Patents Division P.O. Box 68
*Unilever House*
London EC4P 4BQ (GB)

(56) References cited:
PATENTS ABSTRACTS OF JAPAN, vol. 6, no. 57 (C-98)935r, 14th April 1982 & JP-A-57 2 212 (POLA KASEI KOGYO K.K.) 07-01-1982

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to cosmetic compositions in the form of solid sticks, in particular sticks comprising an alkanol, a diol and a soap.

Attempts have been made to develop cosmetic sticks which do not feel waxy when applied to the skin and which deliver active ingredients to the skin, such as deodorant materials, via a vehicle which glides easily over the skin surface and which imparts a cooling sensation to the skin both during and after application.

It has been proposed in EP—A—0024365 (The Procter & Gamble Company) to provide solid cosmetic sticks which include as an essential ingredient an aliphatic polyhydric alcohol having from 2 to 3 carbon atoms and from 2 to 3 hydroxyl groups, examples of which are ethane diol and propane diol. It has also been proposed in Seifen-Ole-Fette-Wasche, Vol. 96 No. 14 July 1980, page 494 to provide solid deodorant sticks containing by weight 8% sodium stearate, 14% ethanol, 37%, 1,3-butylene glycol and 40% 1,2-propylene glycol. Experience has shown, however, that when ethylene glycol is employed, it is extremely difficult to solidify the stick composition and to keep it solid at room temperature, which is the temperature of use. Furthermore, when propane diol is employed, a stick with a relatively wide melting range and a low mean melting point is obtained, which property presents difficulties when manufacturing stick compositions of this type, in that an unnecessarily long cooling time is required to solidify the sticks, and production is accordingly reduced.

It has been discovered that when diols having 2 to 3 carbon atoms, such as ethylene glycol or propane diol are replaced at least partially by a diol, such as butane-1,3-diol, having 4 carbon atoms, then stick compositions having a reduced waxy feel, compared with those containing either of these shorter chain diols exclusively, are obtained. Furthermore, sticks containing butane-1,3-diol have a higher mean melting point and a narrower melting range which reduces processing time during their manufacture so increasing output, and aids in obtaining a stick which in use glides more easily over the skin in view of its non-crystalline structure and uniform texture. It is with cosmetic sticks having these superior properties and with attendant processing advantages that this invention is concerned.

Accordingly, the invention provides a cosmetic gel stick composition which comprises:

i) from 10 to 50% by weight of an alkanol chosen from methanol, ethanol, propan-1-ol and propan-2-ol;

ii) from 10 to 50% by weight of a diol chosen from butane-1,3-diol, butane-1,4-diol and butane-2,3-diol;

iii) from 2 to 15% by weight of a soap chosen from a sodium salt and a potassium salt of fatty acids containing from 14 to 18 carbon atoms, and mixtures thereof; and

iv) from 5 to 70% by weight of a condensation product having the formula:

$$H(C_2H_4O)_a(C_3H_6O)_bOR$$

wherein R is hydrogen or an alkyl group having from 1 to 20 carbon atoms, and a and b are each 0 to 35, a+b not exceeding 35; the composition having an average melting point of from 50 to 65°C and a melting span of no more than 18°C.

In the gel stick composition of the invention the preferred alkanol is ethanol.

The alkanol should form preferably from 20 to 50% by weight of the stick composition.

In the gel stick composition of the invention the preferred diol is butane-1,3-diol.

The cosmetic gel stick compositions of the invention also comprise a soap which functions as a gel-forming agent.

The fatty acid portion of the soap gel-forming agents should be derived from essentially pure saturated or unsaturated higher fatty acids having 14 to 18 carbon atoms. Suitable mixtures of such acids can be employed provided that such mixtures are free from significant proportions of other fatty acids of higher or lower chain length which substantially adversely affect the desired gel-forming properties.

Examples of suitable fatty acids for this purpose include myristic, palmitic, stearic, oleic, linoleic, linolenic, margaric and mixtures of such acids. Naturally occurring sources of such fatty acids include coconut oil, beef tallow, lanolin, fish oil, beeswax, palm oil, peanut oil, olive oil, cottonseed oil, soya bean oil, corn oil, rapeseed oil, rosin acids and greases. Conventional fractionation and/or hydrolysis techniques can be employed, if necessary, to obtain the requisite types of fatty acid from such materials.

Especially preferred fatty acid soap-gel forming agents include sodium stearate, sodium palmitate, potassium stearate, potassium palmitate and sodium myristate. The most preferred gel-forming agent is sodium stearate.

The soap can be provided as a preformed ingredient, or it can be formed during the manufacture of the cosmetic gel stick composition by reaction between one or more appropriate alkalis and one or more appropriate fatty acids as separate ingredients.

The soap should, preferably form from 4 to 10% by weight of the stick composition.

It is apparent that cosmetic gel stick compositions comprising less than 2% by weight of a soap tend to be insufficiently rigid to maintain their structure when in use. Conversely, cosmetic gel stick compositions comprising more than 15% by weight of the soap can exhibit an excessively greasy feel in use.

In the condensation product R is preferably an alkyl group having from 4 to 18 carbon atoms.

Examples of such products are a condensate of 14 moles of propylene oxide with 1 mole butyl alcohol

sold by Union Carbide under the name Fluid AP®, and by British Petroleum under the name Breox B35®; a polypropylene glycol having a molecular weight of 1200; a polyethylene glycol having a molecular weight of 420; a condensate of 20 moles of ethylene oxide and 5 moles of propylene oxide with 1 mole of cetyl alcohol; a condensate of 15 moles of propylene oxide with one 1 mole of stearyl alcohol; and ethylene glycol monomethyl ether. The preferred condensate is Breox B35®.

The condensation product can be present in compositions of the invention at a concentration of preferably from 10 to 70% by weight of the composition.

The cosmetic gel stick compositions of the invention can also contain a variety of optional ingredients suitable for improving composition efficacy, stability and cosmetic appeal. Such optional ingredients include deodorant material, perfumes, dyes, pigments and colouring agents.

A further preferred optional component of compositions according to the invention is a material which helps retard alcohol evaporation and which acts as an antisyneresis agent. Especially preferred materials of this type are cellulose derivatives such as hydroxyalkylcelluloses, such as hydroxypropylcellulose and hydroxyethylcellulose. When present, such alcohol evaporation retarding agents and antisyneresis agents can comprise up to 5%, preferably from 1 to 4% by weight of the composition.

A further optional ingredient of the cosmetic stick composition is a conventional deodorant material. Suitable deodorants include bacteriostatic quaternary ammonium compounds such as cetyltrimethylammonium bromide, cetyl pyridinium chloride, benzethonium chloride, diisobutyl phenoxy ethoxy ethyl dimethyl benzyl ammonium chloride, N-alkylpyridinium chloride, N-cetylpyridinium bromide, sodium N-lauroylsarcosine, sodium N-palmitoylsarcosine, lauroylsarcosine, N-myristoylglycine, potassium N-lauroylsarcosine and stearyltrimethylammonium chloride. When present, deodorants can generally comprise from 0.1 to 1% by weight of the composition.

Other optional ingredients such as perfumes, dyes, pigments and colouring agents, if present, can comprise up to 3% by weight of the stick composition of the invention.

The cosmetic stick composition can also contain up to 20% by weight of water. Anhydrous (i.e. substantially water-free sticks) can be rather hard, and sticks containing more than 20% by weight of water can be too soft to be acceptable in use.

The cosmetic stick composition also have a melting span of no more than 18°C, preferably from 10 to 15°C.

The melting span is the difference between the temperature at which the stick composition melt begins to set and the temperature at which the stick composition is fully solidified, i.e. the melting range, when the composition in a molten state is cooled to the solid state.

Cosmetic gel stick compositions having a melting span in excess of 18°C will require an unduly lengthy cooling time during manufacture, with the consequence that the production rate will be relatively slow and the product consequently more expensive to produce than if a narrower melting span was applicable.

The cosmetic stick composition of the invention should also have an average melting point of from 50 to 65°C. Ideally, the stick composition should have an average melting point of about 55°C.

The average melting point, also known as the drop point, is the average of the melting range and can be determined by the method designated British Standards BS 894: 1956 and BP (1958) Appl. 1Va II pages 823 to 826.

Cosmetic gel stick compositions having an average melting point of less than 50°C are likely to be too soft in use and may not retain their shape, particularly at high ambient temperatures. Also, cosmetic gel stick compositions having an average melting point of greater than 65°C may be too hard, and may not slip easily over the skin surface when in use.

The cosmetic gel stick compositions of the invention can be made by combining the ingredients in liquid form, heating as necessary, and pouring the liquid mixture into a mould having the desired shape and allowing it to set to form sticks.

The cosmetic gel stick compositions can be used by the consumer by rubbing the stick on the area of the body where application is desired. In the case of a deodorant stick, the stick is rubbed, for example, in the axilla area to apply a deodorant agent contained in the stick.

Experiment

As stated earlier in this specification, it was found that diols other than those having 4 carbon atoms, such as the butane diols, were unsuitable for making cosmetic gel compositions.

This was concluded from a comparative experiment in which several similar diols and other polyhydric alcohols were employed in the manufacture of otherwise similar stick compositions, the average melting point and melting span in each case being determined.

The cosmetic sticks in this experiment had the following basic formulation:

| | % w/w |
|---|---|
| Ethanol | 49 |
| Selected diol or polyhydric alcohol | 37 |
| Sodium stearate | 7 |
| Minor ingredients | to 100 |

It was found that when hexane diol, glycerine, ethane diol and polyethylene diol 400 (also known as polyethylene glycol 400) were each tested in this way, the average melting point in each case was less than 40°C. The sticks so obtained were too soft to be stored and used effectively at any but low ambient temperatures. When, however, butane diol was employed, the stick so obtained had a satisfactory average melting point of about 52°C.

The stick containing butane diol also had a narrow melting span of about 10°C, which resulted in a shorter setting time and a higher product output with consequent reduction in unit cost, compared with that of sticks containing the other diols or polyhydric alcohols, each of which had melting spans in excess of 20°C.

Examples 1 to 5

Given below are examples of compositions within the scope of the present invention.

| Ingredient | I<br>% | II<br>% | III<br>% | IV<br>% |
|---|---|---|---|---|
| Ethanol | 39.0 | 49.0 | 49.0 | 49.0 |
| Butane-1,3-diol | 20.0 | 10.0 | 20.0 | 5.0 |
| Breox B35 | 10.0 | 10.0 | 10.0 | 15.0 |
| Sodium stearate | 7.0 | 7.0 | 7.0 | 7.0 |
| Propylene glycol | 17.0 | 17.0 | 7.0 | 17.0 |
| Other ingredients including perfume | up to 100 | up to 100 | up to 100 | up to 100 |
| Melting range (°C) | 45—63 | 41—58 | 57—67 | 45—58 |
| Melting span (°C) | 18 | 17 | 10 | 13 |
| Mean melting point (°C) | 54 | 50 | 62 | 52 |

The above mentioned formulations were made by mixing the ingredients and heating together under reflux at 70°C. In each case the formulation was poured into stick moulds at 70°C and allowed to cool. When the sticks had set at room temperature, they were tested by a panel of trained experts and found to provide deodorant sticks of excellent rheological properties which were pleasant to use.

It should be noted that in each case the average melting point was at least 50°C and in one case 62°C, and that the melting span fell between the values of 10°C and 18°C.

In a comparative example which is not within the scope of the present invention, a stick was prepared containing 49% by weight ethanol, and 20% by weight of Breox® B35, but without any butane-1,3-diol, the remaining ingredients including sodium stearate, propylene glycol and other ingredients being those stated in Examples I to IV, and it was found that this particular formulation yielded a stick with an average melting point of 48°C, a melting range of from 40 to 55°C and a melting span of 15°C. This particular gel stick composition was too soft to be of any real value and was discarded.

**Claims**

1. A cosmetic gel stick composition which comprises:
i) from 10 to 50% by weight of an alkanol chosen from methanol, ethanol, propan-1-ol and propan-2-ol;
ii) from 10 to 50% by weight of a diol chosen from butane-1,3-diol, butane-1,4-diol and butane-2,3-diol;
iii) from 2 to 15% by weight of a soap chosen from a sodium salt and a potassium salt of fatty acids containing from 14 to 18 carbon atoms, and mixtures thereof; and

iv) from 5 to 70% by weight of a condensation product having the formula:

$$H(C_2H_4O)_a(C_3H_6O)_bOR$$

wherein R is hydrogen or an alkyl group having from 1 to 20 carbon atoms, and a&b are each 0 to 35, a+b not exceeding 35; the composition having an average melting point of from 50 to 65°C and a melting span of no more than 18°C.

2. A stick composition according to claim 1, in which the soap is chosen from sodium stearate, sodium palmitate, sodium myristate, potassium stearate and potassium palmitate.

3. A stick composition according to claim 1 or 2, in which the soap forms from 4 to 10% by weight.

4. A cosmetic gel stick composition which comprises:
i) from 39 to 49% by weight of ethanol;
ii) from 5 to 20% by weight of butane-1,3-diol;
iii) from 7 to 17% by weight of propylene glycol;
iv) 7% by weight of sodium stearate; and
v) from 10 to 15% by weight of a propylene oxide condensation product having the formula:

$$H(C_3H_6O)_{14}OC_4H_9OH$$

5. A process for preparing a cosmetic gel stick composition according to claims 1—4 which comprises the steps of:

i) forming a melt of an alkanol chosen from ethanol, propan-1-ol and propan-2-ol; a diol chosen from butane-1,3-diol, butane-1,4-diol and butane-2,3-diol; optionally propylene glycol; a soap chosen from sodium and/or potassium salts of fatty acids containing from 14 to 18 carbon atoms, and mixtures thereof; and a condensation product having the formula:

$$H(C_2H_4O)_a(C_3H_6O)_bOR$$

wherein a, b and R are as defined in claim 1, and

ii) pouring the melt so formed into moulds and allowing the melt to solidify to form cosmetic gel sticks having an average melting point of from 50 to 65°C and a melting span of no more than 18°C.

**Patentansprüche**

1. Kosmetisches Präparat in Form eines Gel-Stiftes, dadurch gekennzeichnet, dass es
i) 10 bis 50 Gew.% eines Alkanols ausgewählt aus Methanol, Aethanol, Propan-1-ol und Propan-2-ol;
ii) 10 bis 50 Gew.% eines Diols ausgewählt aus Butan-1,3-diol, Butan-1,4-diol und Butan-2,3-diol;
iii) 2 bis 15 Gew.% einer Seife ausgewählt aus einem Natriumsalz und einem Kaliumsalz von Fettsäuren enthaltend 14 bis 18 Kohlenstoffatome, und Mischungen davon; und
iv) 5 bis 70 Gew.% eines Kondensationsprodukts der Formel

$$H(C_2H_4O)_a(C_3H_6O)_bOR$$

worin R Wasserstoff oder eine Alkylgruppe mit 1 bis 20 Kohlenstoffatomen und a&b je 0 bis 35 sind aber a+b 35 nicht übersteigt; enthält, wobei das Präparat einen Durchschnittsschmelzpunkt von 50 bis 65°C und einen Schmelzbereich von nicht mehr als 18°C aufweist.

2. Stift-Präparat nach Anspruch 1, dadurch gekennzeichnet, dass die Seife ausgewählt ist aus Natriumstearat, Natriumpalmitat, Natriummyristat, Kaliumstearat und Kaliumpalmitat.

3. Stift-Präparat nach Anspruch 1 oder 2, dadurch gekennzeichnet, dass die Seife 4 bis 10 Gew.% ausmacht.

4. Kosmetisches Präparat in Form eines Gel-Stiftes, dadurch gekennzeichnet, dass es
i) 39 bis 49 Gew.% Aethanol;
ii) 5 bis 20 Gew.% Butan-1,3-diol;
iii) 7 bis 17 Gew.% Propylenglykol;
iv) 7 Gew.% Natriumstearat, und
v) 10 bis 15 Gew.% eines Propylenoxyd-Kondensationsproduktes der Formel

$$H(C_3H_6O)_{14}OC_4H_9OH$$

enthält.

5. Verfahren zur Herstellung eines kosmetischen Präparates in Form eines Gel-Stifts nach den Ansprüchen 1—4, gekennzeichnet durch die Schritte:

i) Bildung einer Schmelze aus einem Alkanol ausgewählt aus Aethanol, Propan-1-ol und Propan-2-ol; einem Diol ausgewählt aus Butan-1,3-diol, Butan-1,4-diol und Butan-2,3-diol; gegebenenfalls

Propylenglykol; einer Seife ausgewählt aus Natrium- und/oder Kaliumsalzen von Fettsäuren mit 14 bis 18 Kohlenstoffatomen, und Mischungen davon; und einem Kondensationsprodukt der Formel

$$H(C_2H_4O)_a(C_3H_6O)_bOR$$

worin a, b und R wie in Anspruch 1 definiert sind, und

ii) Giessen der so gebildeten Schmelze in Formen und Verfestigenlassen der Schmelze unter Bildung kosmetischer Gel-Stifte mit einem Durchschnittsschmelzpunkt von 50 bis 65°C und einem Schmelzbereich von nicht mehr als 18°C.

**Revendications**

1. Composition pour bâton de gel cosmétique, qui comprend:

i) de 10 à 50% en poids d'un alcanol choisi parmi le méthanol, l'éthanol, le propanol-1 et le propanol-2;

ii) de 10 à 50% en poids d'un diol choisi parmi le butane-1,3-diol, le butane-1,4-diol et le butane-2,3-diol;

iii) de 2 à 15% en poids d'un savon choisi parmi un sel de sodium et un sel de potassium d'acides gras contenant de 14 à 18 atomes de carbone, et leurs mélanges; et

iv) de 5 à 70% en poids d'un produit de condensation répondant à la formule:

$$H(C_2H_4O)_a(C_3H_6O)_bOR$$

dans laquelle R est un hydrogène ou un groupe alkyle ayant de 1 à 20 atomes de carbone, et a et b sont chacun de 0 à 35, a+b ne dépassant pas 35; la composition ayant un point de fusion moyen de 50 à 65°C et un intervalle de fusion ne dépassant pas 18°C.

2. Composition pour bâton selon la revendication 1, dans laquelle le savon est choisi parmi le stéarate de sodium, le palmitate de sodium, le myristate de sodium, le stéarate de potassium et le palmitate de potassium.

3. Composition pour bâton selon la revendication 1 ou 2, dans laquelle le savon constitue de 4 à 10% en poids.

4. Composition pour bâton de gel cosmétique, qui comprend:

i) de 39 à 49% en poids d'éthanol;

ii) de 5 à 20% en poids de butane-1,3-diol;

iii) de 7 à 17% en poids de propylène-glycol;

iv) 7% en poids de stéarate de sodium; et

v) de 10 à 15% en poids d'un produit de condensation d'oxyde de propylène ayant pour formule:

$$H(C_3H_6O)_{14}OC_4H_9OH$$

5. Procédé de préparation d'une composition pour bâton de gel cosmétique selon les revendications 1 à 4, qui comprend les étapes qui consistent:

i) à former une masse fondue d'un alcanol choisi parmi l'éthanol, le propanol-1 et le propanol-2; d'un diol choisi parmi le butane-1,3-diol, le butane-1,4-diol et le butane-2,3-diol; éventuellement de propylène-glycol; d'un savon choisi parmi les sels de sodium et/ou de potassium d'acides gras contenant de 14 à 18 atomes de carbone, et de leurs mélanges; et d'un produit de condensation ayant pour formule:

$$H(C_2H_4O)_a(C_3H_6O)_bOR$$

dans laquelle a, b et R sont tels que définis dans la revendication 1 et

ii) à verser la masse fondue ainsi formée dans des moules et à laisser la masse fondue se solidifier pour former les bâtons de gel cosmétiques ayant un point de fusion moyen de 50°C à 65°C et un intervalle de fusion ne dépassant pas 18°C.